# EUROPEAN PATENT SPECIFICATION

(11) **EP 2 034 837 B1**
(45) Date of publication and mention of the grant of the patent: **09.03.2011**
(21) Application number: 07812494.8
(22) Date of filing: 29.06.2007
(51) Int. Cl.: A01N 43/50

(54) **BIOFILM CONTROL**
BIOFILMSTEUERUNG
LUTTE CONTRE UN BIOFILM

(30) Priority: 29.06.2006 US 817384 P
(43) Date of publication of application: 18.03.2009
(73) Proprietor: Albemarle Corporation, Baton Rouge, LA 70801-1765 (US)
(72) Inventor: SAUER, Joe, D., Baton Rouge, LA 70816 (US); NALEPA, Christopher, J., Zachary, LA 70791 (US); PICKRELL, William, S., Baton Rouge, LA 70817 (US); STARRETT, Richmond, M., Baton Rouge, LA 70810 (US); ELNAGAR, Hassan, Y., Baton Rouge, LA 70810 (US); COOK, George, W., Jr., Baton Rouge, LA 70809 (US); MOORE, Robert, M., Jr., - (US)
(74) Representative: UEXKÜLL & STOLBERG
(86) International application number: PCT/US2007/072516
(87) International publication number: WO 2008/003085

(56) References cited:
- WO-A-2004/060818
- US-B2- 6 638 959

## Description

### BACKGROUND

Biofilms are mucilaginous communities of microorganisms such as bacteria, archaea, fungi, molds, algae or protozoa or mixtures thereof that grow on various surfaces. Biofilms form when microorganisms establish themselves on a surface and activate genes involved in producing a matrix that includes polysaccharides. This matrix may provide protection of biofilm bacteria from biocides.

Molecules called quorum-sensing signals help trigger and coordinate part of the process of forming a biofilm via cell-to-cell communication/signalling. Bacteria constantly secrete low levels of the signals and sense them either through receptors on their surfaces, or internally. The receptors trigger behavioral changes when there are enough bacteria to allow the signals' concentrations to achieve a critical threshold. Once this occurs, bacteria respond by adopting communal behavior, such as forming a biofilm, and in the case of pathogenic bacteria, deploying virulence factors such as toxins. In addition to communicating with members of their own species, bacteria also conduct inter-species communications, such that a biofilm may contain more than one species of bacteria.

For example, the bacteria *Pseudomonas aeruginosa* is widely distributed in water, and also in soil, sewage and plants, *Pseudomonas aeruginosa* use acylated homoserine lactones (HSL) as cell-to-cell signals to control various functions of their communal environment, e.g., to control biofilms harboring them. One identified HSL signal for *Pseudomonas aeruginosa* is 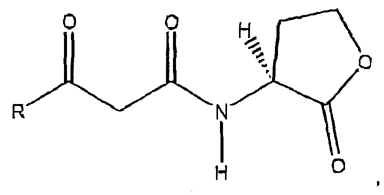 which represents an "attachment" signal for *Pseudomonas aeruginosa* when R is a propyl group. Another identified HSL signal for *Pseudomonas aeruginosa* is: 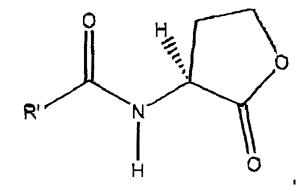 , which represents a "detachment" signal for *Pseudomonas*
*aeruginosa* when R' is a propyl group.

Biofilms can develop into macroscopic structures several millimeters or centimeters in thickness and cover large surface areas. For non-living objects, these formations can play a role in restricting or entirely blocking flow in plumbing systems, decreasing heat transfer in heat exchangers, or causing pathogenic problems in municipal water supplies, food processing, medical devices (e.g., catheters, orthopedic devices, implants). Moreover, biofilms often decrease the life of materials through corrosive action mediated by the embedded microorganisms. This biological fouling is a serious economic problem in industrial water process systems, pulp and paper production processes, cooling water systems, injection wells for oil recovery, cooling towers, porous media (sand and soil), marine environments, and air conditioning systems, and any closed water recirculation system. Biofilms are also a severe problem in medical science and industry, said to cause dental plaque, infections, contaminated endoscopes and contact lenses, prosthetic device colonisation and biofilm formation on medical implants.

In other areas, biofilms can be quite useful. For example, biofilms are an integral part of the human body and are especially beneficial In protecting the intestine from attack by harmful bacteria. One of the best examples of beneficial application of biofilms to solve a major problem is in the cleaning of wastewater. Biofilms can also be used to clean up oil and gasoline spills. Bioremediation using biofilms has emerged as an optional technology for cleaning up groundwater at many sites containing hazardous waste.

Numerous publications describe compositions and methods directed at treating water in an effort to destroy biofilms and the bacteria they harbor. Dimethylhydantoin (DMH) is used in some halogenated biocidal water treatment products. US Patent 6638959 describes the use of dibromodialkylhydantoin (DBDAH). e.g., dibromodimethythydantoin (DBDMH) to treat water that is in contact with, or that comes into contact with, biofilms.

Further, WO2004/060818 discloses a method of disintegrating biofilm, flocculent bulked sludge or bulked biologically active sludge in an aqueous system, which comprises adding to or forming in said aqueous medium containg biofilm, flocculent bulked sludge or bulked biologically active sludge one or more chlorinated hydantoins. The chlorinated hydantoin may be added as such or may be formed from alkylated hydantoins.

In spite of the current body of knowledge regarding biofilms and cell-to-cell communication, there remains a need for commercially feasible means of utilizing cell-to-cell communication to control biofilms harboring bacteria and other contaminants, whether the contaminants originated in water or some other medium. Additionally, in spite of the availability of commercial halogenated biocides, there remains a need for improved halogenated biocides with enhanced biofilm controlling capabilities.

### THE INVENTION

This invention meets the above-described needs by providing use of 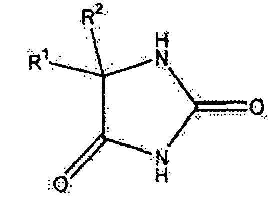 to control a biofilm comprising a microorganism, wherein 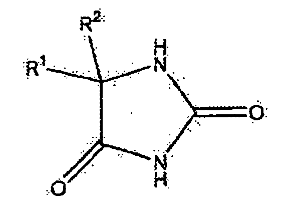 is in contact with the biofilm and emulates a cell-to-cell signal molecule of the microorganism. R¹ comprises a hydrogen atom or a hydrocarbyl radical having from 1 to about 14 carbon atoms and R² independently comprises a hydrocarbyl radical having from 1 to about 14 carbon atoms. Molecules of this invention are designed to be physically and electrically similar to cell-to-cell signal molecules for microorganisms such that the molecules of this invention emulate the cell-to-cell signal molecules. Also described methods for synthesizing 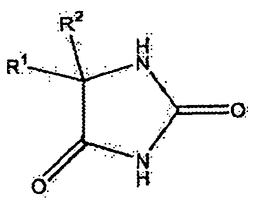 suitable for controlling biofilm, wherein R¹ comprises a hydrogen atom or a hydrocarbyl radical having from 1 to about 14 carbon atoms and R² independently comprises a hydrocarbyl radical having from 1 to about 14 carbon atoms, the methods comprising converting a ketone to a cyanohydrin by combining the ketone with sodium bisulfate, sodium cyanide and ammonium carbonate. Said use for controlling biofilm on substrate in contact with liquid medium, wherein the biofilm comprises a microorganism, the comprises introducing into the liquid medium 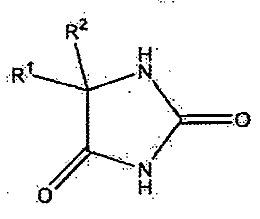 that emulates a cell-to-cell signal molecule of the microorganism, wherein R¹ comprises a hydrogen atom or a hydrocarbyl radical having from 1 to about 14 carbon atoms and R² independently comprises a hydrocarbyl radical having from 1 to about 14 carbon atoms. R¹ and R² can be the same or different. In some embodiments, when R¹ and/or R² comprise a hydrocarbyl radical, the radical has from 1 to about 6, or 3 to 6, carbon atoms.

As used herein: (i) "biofilm" means a mucilaginous community that can grow on various substrates, said community comprised of one or more microorganisms, such as, for example, bacteria, archaea, fungi, molds, algae or protozoa, or the like; (ii) "substrate" means any surface on which a biofilm can form or has formed, and includes, but is not limited to, hard or soft surfaces such as polymers, plastics, tubing, ceramics, metals, glass, hydroxyapatite, skin, bone, tissues, and geological formations such as layers of sand; (iii) "to control/controlling" a biofilm means to cause dissolution of at least a portion of a biofilm, to prevent formation of a biofilm or additional growth of an existing biofilm, and/or to cause a biofilm to form in a more desired manner or location. Cell-to-cell signal molecules for microorganisms comprising a biofilm control the biofilm by sending appropriate signals. For example, a "detachment" signal of a microorganism signals the microorganism to detach from a biofilm and may cause dissolution of at least a portion of the biofilm; a "do not attach" signal or "remain planktonic" signal of a microorganism signals the microorganism to remain planktonic and may prevent formation of a biofilm or additional growth of an existing biofilm; an "attachment" signal of a microorganism signals the microorganism to attach, e.g., to a biofilm, and given at appropriate times and locations may cause a biofilm to form in a more desired manner or location; and a "do not detach" signal or "remain sessile" signal of a microorganism signals a microorganism to remain sessile and may prevent at least a portion of a biofilm from dissolving.

This invention also provides additionnal use of 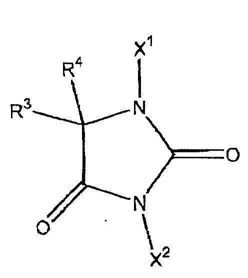 in contact with biofilm comprising a microorganism, wherein 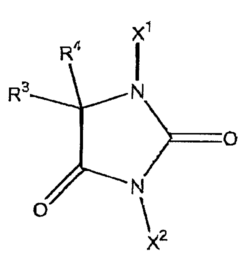 emulates a cell-to-cell signal molecule of the microorganism, R³ comprises a hydrogen atom or a hydrocarbyl radical having from 1 to about 14 carbon atoms, R⁴ independently comprises a hydrocarbyl radical having from 1 to about 14 carbon atoms, and either (i) X¹ comprises a hydrogen atom or a halogen atom and X² independently comprises a halogen atom, or (ii) X² comprises a hydrogen atom or a halogen atom and X¹ independently comprises a halogen atom.

Thus, there is use of a composition to control biofilm comprising one or more microorganisms, wherein the composition is in contact with biofilm and comprises a molecule capable of killing the microorganism and 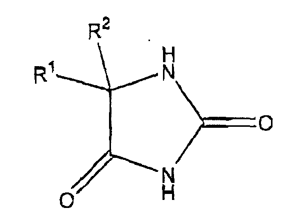 that emulates a cell-to-cell signal molecule of the microorganism, wherein R¹ comprises a hydrogen atom or a hydrocarbyl radical having from 1 to about 14 carbon atoms and R² independently comprises a hydrocarbyl radical having from 1 to about 14 carbon atoms. Said molecule capable of killing the microorganism may be 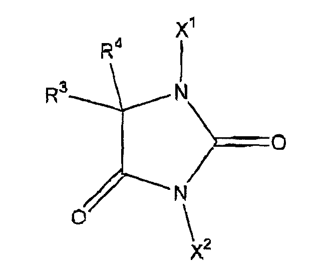 that emulates a cell-to-cell signal molecule of one of the microorganism, wherein R³ comprises a hydrogen atom or a hydrocarbyl radical having from 1 to about 14 carbon atoms, R⁴ independently comprises a hydrocarbyl radical having from 1 to about 14 carbon atoms, and either (i) X¹ comprises a hydrogen atom or a halogen atom and X² independently comprises a halogen atom, or (ii) X² comprises a hydrogen atom or a halogen atom and X¹ independently comprises a halogen atom.

Also described are methods for synthesizing 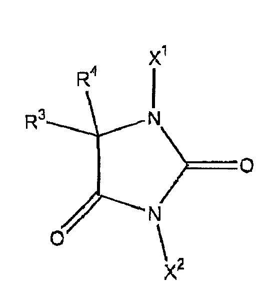 suitable as further component for controlling biofilm, wherein R³ comprises a hydrogen atom or a hydrocarbyl radical having from 1 to about 14. carbon atoms, R⁴ independently comprises a hydrocarbyl radical having from 1 to about 14 carbon atoms, and either (i) X¹ comprises a hydrogen atom or a halogen atom and X² independently comprises a halogen atom, or (ii) X² comprises a hydrogen atom or a halogen atom and X¹ independently comprises a halogen atom, the methods comprising converting a ketone to a cyanohydrin by combining the ketone with sodium bisulfate, sodium cyanide and ammonium carbonate. Said use for controlling biofilm on substrate in contact with liquid medium, wherein the biofilm comprises a microorganism, comprises additionally introducing into the liquid medium as a further component 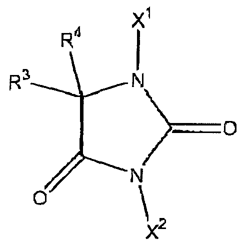 according to this embodiment that emulates a cell-to-cell signal molecule of the microorganism, wherein R³ comprises a hydrogen atom or a hydrocarbyl radical having from 1 to about 14 carbon atoms, R⁴ independently comprises a hydrocarbyl radical having from 1 to about 14 carbon atoms, and either (i) X¹ comprises a hydrogen atom or a halogen atom and X² independently comprises a halogen atom, or (ii) X² comprises a hydrogen atom or a halogen atom and X¹ independently comprises a halogen atom. R³ and R⁴ can be the same or different. In some embodiments, when R³ and/or R⁴ comprise a hydrocarbyl radical, the radical has from 1 to about 6, or 3 to 6, carbon atoms. X¹ and X² can be the same or different.

As mentioned this invention also provides use of compositions in contact with biofilm comprising a microorganism, the compositions comprising a molecule capable of killing the microorgansim and 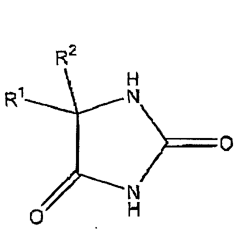 that emulates a cell-to-cell signal molecule of the microorganism, wherein R¹ comprises a hydrogen atom or a hydrocarbyl radical having from 1 to about 14 carbon atoms and R² independently comprises a hydrocarbyl radical having from 1 to about 14 carbon atoms. As used in this embodiment, the molecule capable of killing bacteria can comprise any molecule capable of killing the bacteria, including without limitation dibromodialkylhydantoins
(DBDAH), bromochlorodialkylhydantoins (BCDAH), and 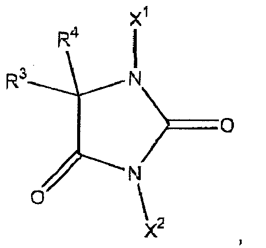 wherein R³ comprises a hydrogen atom or a hydrocarbyl radical having from 1 to about 14 carbon atoms, R⁴ independently comprises a hydrocarbyl radical having from 1 to about 14 carbon atoms, and either (i) X¹ comprises a hydrogen atom or a halogen atom and X² independently comprises a halogen atom, or (ii) X² comprises a hydrogen atom or a halogen atom and X¹ independently comprises a halogen atom. Said use for killing a microorganism such as bacteria, at least a portion of which is harbored in biofilm on substrate in contact with liquid medium, comprises introducing into the liquid medium at least one molecule capable of killing the microorganism and 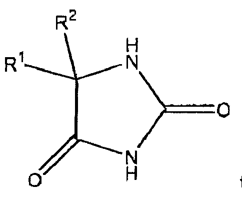 that emulates a cell-to-cell signal molecule of the microorganism, wherein R¹ comprises a hydrogen atom or a hydrocarbyl radical having from 1 to about 14 carbon atoms and R² independently comprises a hydrocarbyl radical having from 1 to about 14 carbon atoms. Each of R¹, R², R³ and R⁴ can be the same or different.

This invention is described in connection with specific embodiments. It is understood that this invention is not limited to any one of these specific embodiments.

In 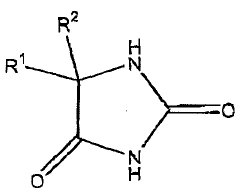 used according to this invention, R¹ and R² are independently selected to have the length and electronics needed to enable the molecule to emulate the desired cell-to-cell signal molecule. For purposes of example only, either or both of R¹ and R² can comprise a carbonyl or an alkyl.
In 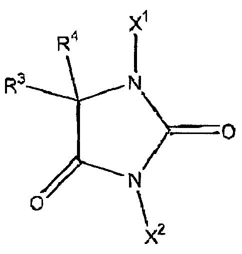 additionally used as further component according to this invention, Ra³ and R⁴ are independently selected to have the length and electronics needed to enable the molecule to emulate the desired cell-to-cell signal molecule. For purposes of example only, either or both of R³ and R⁴ can comprise a carbonyl or an alkyl. When the molecule is intended to kill bacteria, either or both of X¹ and X² can comprise a halogen atom capable of killing the bacteria, including without limitation a bromine atom, a chlorine atom, or an iodine atom.

In 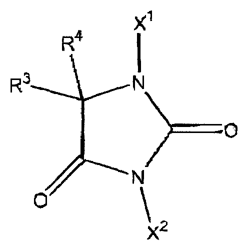 additionally used as further component according to this invention, the hydantoin nucleus retains the ability to stabilize the halogen atom(s) X¹ and/or X² attached to the N atoms within the ring. Depending on the composition of X¹, X², R³, and R⁴, this stabilization can be increased or decreased. When either or both of X¹ and X² is a bromine, the product bromamine is not only capable of holding the halogen and stabilizing it, but it is also capable of releasing it as active HOBr in aqueous solution according to the following acid-based equilibrium reaction:

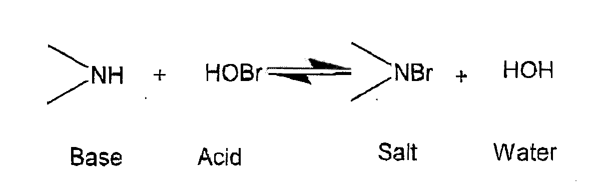

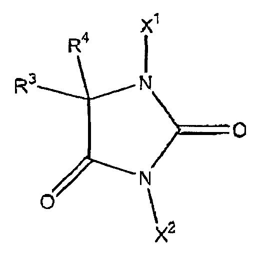

additionally used according to this invention mav be synthesized bv converting a ketone molecule to a molecule having the same ligands groups on the 5-carbon, as were initially present as flanking groups at the ketone carbonyl. Other routes are also possible and should not be considered to be excluded from this invention.

### EXAMPLES

In the molecules used in this invention and illustrated in the following examples, R¹ and R² were selected for size, length, and electronic character, to make molecules that are essentially indistinguishable from legitimate bacterial molecular signals.

### Example 1: 5-Methyl-5-Ethylhydantoin

2-Butanone was converted to an intermediate cyanohydrin (I) by treatment with sodium bisulfite followed by sodium cyanide. The intermediate cyanohydrin (I) was admixed with ammonium carbonate and warmed to form the crude 5-methyl-5-ethylhydantoin (II), which was isolated and purified by standard crystallization and recrystallization operations.

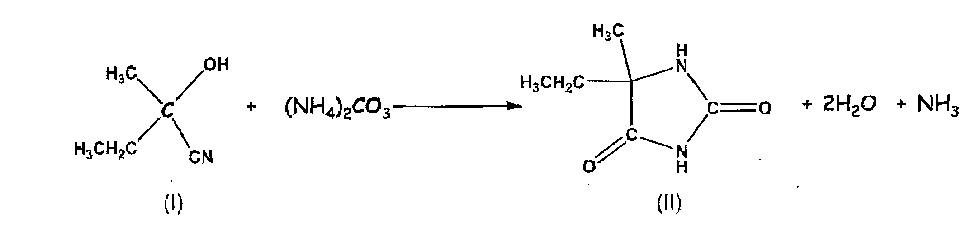

### Example 2: 5-Methyl-5-Propylhydantoin

Using a method similar to that used in Example 1, 2-Pentanone was converted to an intermediate cyanohydrin (III) and finally to the desired 5-methyl-5-propylhydantoin (IV),

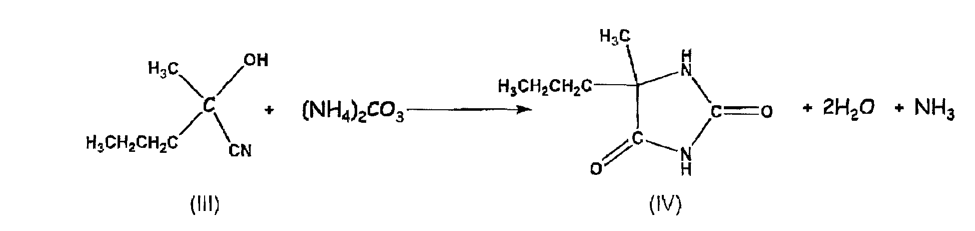

### Example 3: 5,5-Diethylhydantoin

Using a method similar to that used in Example 1, 1, 3-Pentanone was converted to a symmetric, intermediate cyanohydrin and finally to the desired 5,5-diethylhydantoin.

### Example 4: 5-Methyl-5-Heptylhydantoin

Using a method similar to that used in Example 1, 2-Nonanone was converted to the desired product 5-methyl-5-heptylhydantoin.

Composition used according to this invention have the ability to behave as oxidizing biocides, in their own right. For example, bromamines are biocidal. Other embodiments, such as chloramines, while not nearly as effective biocides as bromamines, are still irritants to bacteria, and under some condition may also provide biocidal action. Chlorinated or mixed chlorine and other halogen species are expected to provide slow, or long-term, residual biocidal control effects.

## Claims

1. Use of 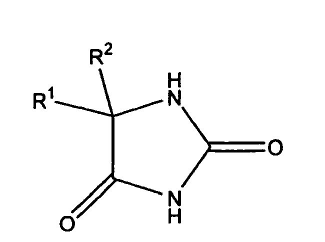 to control a biofilm comprising a microorganism, wherein the 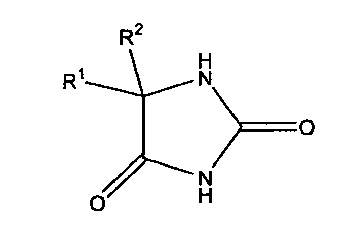 is in contact with the biofilm and emulates a cell-to-cell signal molecule of the microorganism, and wherein R¹ comprises a hydrogen atom or a hydrocarbyl radical having from 1 to about 14 carbon atoms and R² independently comprises a hydrocarbyl radical having from 1 to about 14 carbon atoms.

2. Use of a composition to control biofilm comprising one or more microorganisms, wherein the composition is in contact with biofilm and comprises a molecule capable of killing the microorganism and 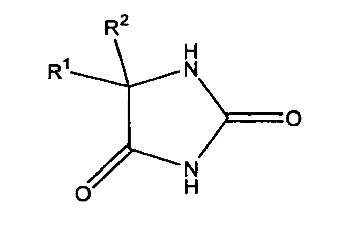 that emulates a cell-to-cell signal molecule of the microorganism, wherein R¹ comprises a hydrogen atom or a hydrocarbyl radical having from 1 to about 14 carbon atoms and R² independently comprises a hydrocarbyl radical having from 1 to about 14 carbon atoms.

3. Use of claim 2 wherein said molecule capable of killing the microorganism is 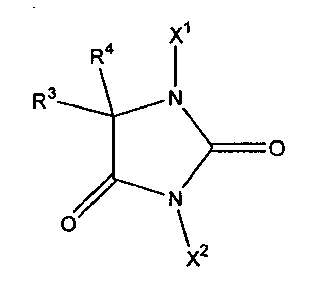 that emulates a cell-to-cell signal molecule of one of the microorganisms, wherein R³ comprises a hydrogen atom or a hydrocarbyl radical having from 1 to about 14 carbon atoms, R⁴ independently comprises a hydrocarbyl radical having from 1 to about 14 carbon atoms, and either (i) X¹ comprises a hydrogen atom or a halogen atom and X² independently comprises a halogen atom, or (ii) X² comprises a hydrogen atom or a halogen atom and X¹ independently comprises a halogen atom.

## Patentansprüche

1. Verwendung von 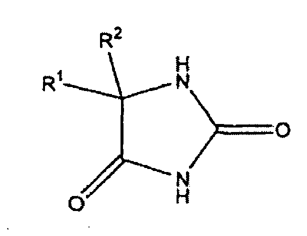 zum Kontrollieren eines Biofilms, der einen Mikroorganismus enthält, bei der das 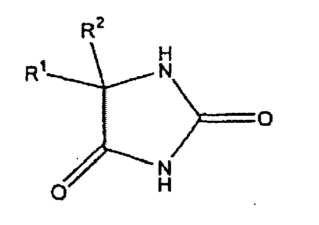 in Kontakt mit dem Biofilm ist und ein Zelle-zu-Zelle-Signalmolekül des Mikroorganismus nachahmt, wobei R¹ ein Wasserstoffatom oder einen Kohlenwasserstoffrest mit 1 bis etwa 14 Kohlenstoffatomen umfasst und R² unabhängig einen Kohlenwasserstoffrest mit 1 bis etwa 14 Kohlenstoffatomen umfasst.

2. Verwendung einer Zusammensetzung zum Kontrollieren eines Biofilms, der einen oder mehrere Mikroorganismen enthält, bei der die Zusammensetzung in Kontakt mit dem Biofilm ist und ein Molekül, das dazu in der Lage ist, den Mikroorganismus abzutöten, und 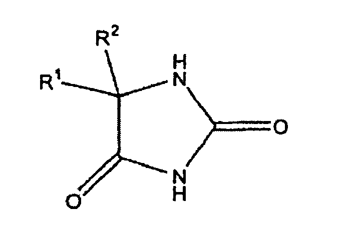 enthält, das ein Zelle-zu-Zelle-Signalmolekül des Mikroorganismus nachahmt, wobei R¹ ein Wasserstoffatom oder einen Kohlenwasserstoffrest mit 1 bis etwa 14 Kohlenstoffatomen umfasst und R² unabhängig einen Kohlenwasserstoffrest mit 1 bis etwa 14 Kohlenstoffatomen umfasst.

3. Verfahren nach Anspruch 2, bei dem das Molekül, das dazu in der Lage ist, den Mikroorganismus abzutöten, 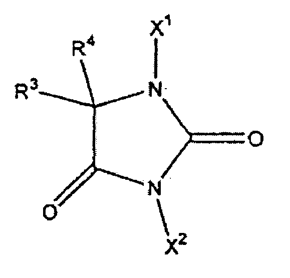 ist, das ein Zelle-zu-Zelle-Signalmolekül eines der Mikroorganismen nachahmt, wobei R³ ein Wasserstoffatom oder einen Kohlenwasserstoffrest mit 1 bis etwa 14 Kohlenstoffatomen umfasst, R⁴ unabhängig einen Kohlenwasserstoffrest mit 1 bis etwa 14 Kohlenstoffatomen umfasst, und entweder (i) X¹ ein Wasserstoffatom oder ein Halogenatom umfasst und X² unabhängig ein Halogenatom umfasst, oder (ii) X² ein Wasserstoffatom oder ein Halogenatom umfasst und X¹ unabhängig ein Halogenatom umfasst.

## Revendications

1. Utilisation de 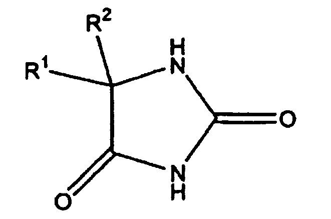 pour contrôler un biofilm comprenant un microorganisme, dans laquelle le 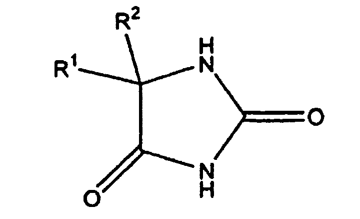 est en contact avec le biofilm et imite une molécule de signal de cellule à cellule du microorganisme, et dans laquelle R¹ comprend un atome d'hydrogène ou un radical hydrocarbyle ayant de 1 à environ 14 atomes de carbone et R² comprend indépendamment un radical hydrocarbyle ayant de 1 à environ 14 atomes de carbone.

2. Utilisation d'une composition pour contrôler un biofilm comprenant un ou plusieurs microorganismes, dans laquelle la composition est en contact avec le biofilm et comprend une molécule capable de tuer le microorganisme et 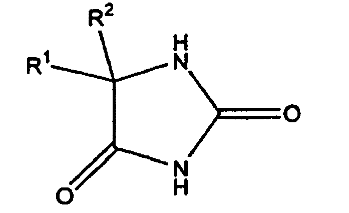 qui imite une molécule de signal de cellule à cellule du microorganisme, dans laquelle R¹ comprend un atome d'hydrogène ou un radical hydrocarbyle ayant de 1 à environ 14 atomes de carbone et R² comprend indépendamment un radical hydrocarbyle ayant de 1 à environ 14 atomes de carbone.

3. Utilisation selon la revendication 2, dans laquelle ladite molécule capable de tuer le microorganisme est 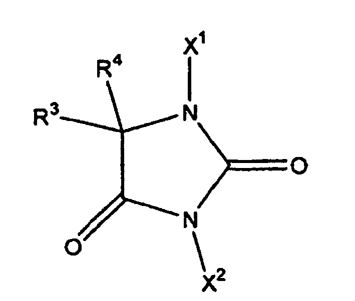 qui imite une molécule de signal de cellule à cellule de l'un des microorganismes, dans laquelle R³ comprend un atome d'hydrogène ou un radical hydrocarbyle ayant de 1 à environ 14 atomes de carbone, R⁴ comprend indépendamment un radical hydrocarbyle ayant de 1 à environ 14 atomes de carbone, et soit (i) X¹ comprend un atome d'hydrogène ou un atome d'halogène et X² comprend indépendamment un atome d'halogène, soit (ii) X² comprend un atome d'hydrogène ou un atome d'halogène et X¹ comprend indépendamment un atome d'halogène.
